(19) Europäisches Patentamt
European Patent Office
Office européen des brevets

(11) Numéro de publication: **0 480 841 B1**

(12) # FASCICULE DE BREVET EUROPEEN

(45) Date de publication du fascicule du brevet: **27.12.95**

(51) Int. Cl.[6]: **A01N 33/08**, A01N 33/24, //(A01N33/08,33:24,25:30), (A01N33/24,33:08,25:30)

(21) Numéro de dépôt: **91402722.2**

(22) Date de dépôt: **11.10.91**

(54) **Composition désinfectante pour l'industrie agro-alimentaire et la médecine vétérinaire**

(30) Priorité: **11.10.90 FR 9012567**

(43) Date de publication de la demande:
**15.04.92 Bulletin 92/16**

(45) Mention de la délivrance du brevet:
**27.12.95 Bulletin 95/52**

(84) Etats contractants désignés:
**BE CH DE ES IT LI NL**

(56) Documents cités:
**FR-A- 792 822**
**FR-A- 2 301 235**
**FR-A- 2 406 439**

(73) Titulaire: **C F P I**
**28 Boulevard Camélinat,**
**Boîte Postale 75**
**F-92233 Gennevilliers Cédex (FR)**

(72) Inventeur: **Schapira, Joseph**
**32 Rue Miollis**
**F-75015 Paris (FR)**
Inventeur: **Vincent, Jacques**
**21 Rue des Closeaux**
**F-78750 Mareil Marly (FR)**
Inventeur: **Gamet, Jean Claude**
**36 Rue Paul Vaillant Couturier**
**F-18400 St Florent Sur Chair (FR)**
Inventeur: **Geneix, Catherine**
**89 Rue des Alouettes**
**F-92000 Nanterre (FR)**

(74) Mandataire: **Koch, Gustave et al**
**Cabinet PLASSERAUD**
**84, rue d'Amsterdam**
**F-75440 Paris Cédex 09 (FR)**

**EP 0 480 841 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

L'invention a pour objet une composition désinfectante pour l'industrie agro-alimentaire et la médecine vétérinaire.

Les agents bactéricides de formule:

$$R - X - CH_2 - CHOH - CH_2NH_2 \quad (I)$$

dans laquelle

- X représente -O-, -S-, -NH- ou $-CH_2-$,
- R représente un radical aliphatique à chaîne droite en $C_5$ à $C_{18}$ et est différent de $C_{12}$ lorsque X est un radical $-CH_2-$,

ainsi que leurs sels, esters et sels d'alcoyle ammonium quaternaire, ont déjà été utilisés en tant qu'agents stérilisants et sont décrits dans le brevet FR-A-2 301 235.

Ils donnent généralement satisfaction.

Mais, devant les exigences de plus en plus grandes des utilisateurs, la Société Demanderesse a effectué des recherches pour mettre au point des produits plus performants.

Et elle a eu le mérite de trouver que, de façon inattendue et surprenante, une combinaison d'un agent bactéricide de formule (I) et d'un agent tensio-actif non ionique de formule:

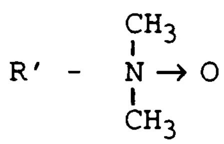

(II)

dans laquelle R' représente un radical aliphatique en $C_{12}$ à $C_{16}$, présente une activité bactéricide supérieure à la somme des activités bactéricides des deux agents pris isolément.

Les agents de formule (II) ont déjà été décrits dans les brevets FR-A-792 822 et FR-A-2 406 439 mais leur combinaison avec un agent bactéricide de formule (I) n'a pas été envisagée dans ces deux documents.

Il s'ensuit que la composition désinfectante conforme à l'invention est caractérisée par le fait qu'elle comporte, en tant que matière active, en combinaison,

- d'une part, un agent bactéricide de formule (I) et,
- d'autre part, un agent tensio-actif non ionique de formule (II),

étant entendu que ladite composition comporte les agents de formules (I) et (II) en un rapport pondéral II/I compris entre 5 et 0,5.

Selon un mode de réalisation avantageux, la susdite composition comprend en combinaison,

- a titre d'agent bactéricide, le décyloxy-3-hydroxy-2-amino-1-propane en particulier sous forme de chlorhydrate et,
- à titre d'agent tensio-actif non ionique, le N,N'-diméthyldodécylamine-N-oxyde.

Dans la pratique, l'agent tensio-actif non ionique est un mélange de composés de formule (II) comportant une très forte proportion, notamment supérieure à 65% en poids, de N,N'-diméthyldodécylamine-N-oxyde.

Selon un autre mode de réalisation avantageux, la susdite composition contient de 50 à 2% en poids de la combinaison d'agents de formules (I) et (II).

Selon un autre mode de réalisation avantageux, la susdite composition contient les agents de formules (I) et (II) en un rapport pondéral II/I compris entre 3 et 1.

Selon un autre mode de réalisation avantageux, la susdite composition est appliquée à la désinfection dans l'industrie laitière, à la ferme, dans les abattoirs, dans les installations de fabrication de charcuterie et de boissons.

La susdite composition peut être utilisée pour la prévention de la mammite subclinique chez les ruminants, notamment chez la vache, par application sur le pis avant et/ou après la traite.

Dans le cas de son utilisation pour la prévention de la mammite subclinique, la susdite composition peut contenir, outre la combinaison des agents de formules (I) et (II),

- au moins un agent tensio-actif,
- au moins un agent émollient à effet nourrissant, adoucissant et cicatrisant et
- au moins un agent filmogène et/ou épaississant, la formulation pouvant être complétée par de l'eau et/ou par au moins un alcool et/ou par un colorant et/ou par un parfum.

Dans le cas de la prévention de la mammite, on utilise un produit pur, qui sera donc peu chargé en matières actives. Par contre en application industrielle, on utilise un produit commercial qui est dilué au moment de l'emploi et qui est donc beaucoup plus riche en principes actifs.

L'invention pourra être encore mieux comprise à l'aide du complément de description qui suit et des exemples non limitatifs dans lesquels sont illustrés des modes de réalisation avantageux.

Pour montrer que l'activité désinfectante de la combinaison des agents de formule (I) et (II) est supérieure à la somme des activités de chacun des deux agents pris isolément, on a préparé

- une première composition A constituée par une solution dans l'eau à pH 7 (à l'aide de NaOH) à 1% en poids de chlorhydrate de décyloxy-3-hydroxy-2-amino-1-propane,
- une deuxième composition B constituée par une solution dans l'eau à pH 7 (à l'aide de NaOH) à 1,8% en poids de diméthyldodécylamine-oxyde sous la forme du produit commercialisé par la Société AKZO sous l'appellation "AROMOX DMMCD-W" qui est une solution aqueuse à 30% d'un mélange de trois agents de formule (II), à savoir respectivement 68% d'agent en $C_{12}$, 29% d'agent en $C_{14}$ et 3% d'agent en $C_{10}$,
- une troisième composition C constituée par une solution dans l'eau à pH 7 (à l'aide de NaOH) à respectivement 1% en poids du produit actif de la composition A et 1,8% en poids du produit actif de la composition B.

Pour tester l'activité bactéricide des trois compositions, on les fait agir sur des cultures de S. aureus et de P. aeruginosa.

La méthode d'analyse retenue pour déterminer l'activité bactéricide des produits consiste à déterminer la concentration minimale capable de diminuer la population bactérienne de ces souches dans une proportion au moins égale à 100.000 fois après 5 minutes de contact à 21°C (norme AFNOR NFT 72150/1); cette concentration est désignée par l'expression "concentration minimale bactéricide" ou CMB.

Cela suppose le dénombrement par les techniques classiques de bactériologie des populations bactériennes initiales et finales; par ailleurs, il est nécessaire de disposer d'un produit de neutralisation des compositions désinfectantes permettant de limiter son action à 5 minutes et n'agissant pas, en ce qui le concerne, sur les bactéries. Un tel produit peut être constitué par un mélange de lécithine d'oeuf 5% (P/V), de Tween 80 3% (P/V) et de thiosulfate de sodium 0,5% (P/V).

Les résultats enregistrés pour les compositions A, B et C sont réunis dans le tableau I.

## TABLEAU I

| Composition | CMB (% V/V) | |
|---|---|---|
| | S. aureus | P. aeruginosa |
| A | 7,5 | 1 |
| B | 2,5 | inactif à 90% |
| C | 1 | 0,5 |

Ces résultats montrent bien l'efficacité de la composition conforme à l'invention.

On a également démontré que l'activité bactéricide est fonction du pH de la composition.

A cet efet, on a testé la composition C aux pH de 7, 6 et 5 en ajustant ledit pH aux valeurs en question à l'aide de NaOH et d'acide acétique.

Les résultats sont réunis dans le tableau II.

**TABLEAU II**

| Composition | pH | CMB (% V/V) Staphylococcus aureus |
|---|---|---|
| C | 7 | 1 |
| C | 6 | ≤ 1 |
| C | 5 | 5 |

Il n'y a donc pas intérêt à abaisser le pH en dessous de 6.

Dans la pratique, les surfaces devant être désinfectées sont fréquemment souillées par des matières notamment protéiques dites "interférentes"; et il est connu que de nombreuses substances désinfectantes voient leur activité diminuer en présence de ces dernières et ceci est notamment le cas pour le chlorhydrate de décyloxy-3-hydroxy-2-amino-1-propane.

De façon surprenante et inattendue, cet inconvénient est supprimé dans le cas de la composition conforme à l'invention et c'est là un avantage important.

Pour illustrer cet avantage, on a testé la composition A et la composition C sur Staphylococcus aureus et sur Streptococcus dysgalactiae en l'absence et en présence d'une matière interférente constituée par un mélange comportant en milieu neutre (pH = 6,8-7) une quantité de 1% (P/V) d'albumine bovine et 1% (P/V) d'extrait de levure (norme AFNOR NFT 72170/1).

Les résultats sont réunis dans le tableau III.

**TABLEAU III**

| Composition | Mélange interférent | CMB (% V/V) | |
|---|---|---|---|
| | | S.aureus | S.dysgalactiae |
| A | absent | 7,5 | 5 |
| A | présent | 30 | 5 |
| C | présent | 2,5 | 2,5 |

Ces résultats font bien apparaître le susdit avantage.

Pour déterminer les limites entre lesquelles doit être choisi, de préférence, le rapport pondéral "agent non ionique/agent bactéricide", il a été procédé aux expériences résumées par le tableau IV.

TABLEAU IV

| Expérience No. | Composition utilisée | | | pH (par NaOH) | CMB (% V/V) | |
|---|---|---|---|---|---|---|
| | diméthyl-dodécyl-amine oxyde | chlorhydrate de décyloxy-3-hydroxy-2-amino-1-propane | eau q.s.p. 100 | | S. aureus | P. aeruginosa |
| 1 | 0 | 1 | 99 | 7 | 7,5 | 1 |
| 2 | 0,5 | 1 | 98,5 | 7 | 5 | |
| 3 | 1,8 | 0 | 98,2 | 7 | 2,5 | inactif à 90% |
| 4 | 1,8 | 1 | 97,2 | 7 | 1 | 0,5 |
| 5 | 3 | 1 | 96 | 7 | 0,5 | |
| 6 | 5 | 1 | 94 | 7 | 0,5 | |

Il résulte des éléments réunis dans ce tableau qu'il ne sert à rien d'augmenter indéfiniment le rapport agent non ionique/agent bactéricide.

Ce rapport sera donc choisi compte tenu des susdits résultats entre 5 et 0,5, de préférence entre 3 et 1.

Le caractère inattendu et surprenant de la potentialisation de l'effet de l'agent bactéricide à l'aide du composé de formule (II) a été démontré à l'aide des essais comparatifs suivants dans lesquels on a remplacé le N,N-diméthylalcoylamine-oxyde par deux autres agents tensio-actifs non ioniques et par deux agents tensio-actifs amphotères.

Les deux agents tensio-actifs amphotères testés sont:

A: la cocoamidopropyl bétaïne

B: la cocoamidopropylhydroxysultaïne.

Les deux autres agents tensio-actifs non ioniques testés sont:

C: le nonylphénol à dix molécules d'oxyde d'éthylène

D: alcool gras éthoxylé sur base alcool $C_9$-$C_{11}$ à 6 molécules d'oxyde d'éthylène.

Dans les expériences comparatives qui suivent, l'agent non ionique utilisé conformément à l'invention, à savoir la N,N-diméthyldodécylamine-N-oxyde, est désigné par E.

On a déterminé la CMB comme dans les expériences précédentes.

Les résultats sont réunis dans le tableau V.

TABLEAU V

| Expérience No. | Composition utilisée | | | | | | eau q.s.p. 100 | pH (par NaOH) | CMB (% V/V) |
|---|---|---|---|---|---|---|---|---|---|
| | chlorhydrate de décyloxy-3-hydroxy-2-amino-1-propane | A | B | C | D | E | | | S. aureus |
| 7 | 1 | 0 | 0 | 0 | 0 | 0 | 99 | 7 | 7.5 |
| 8 | 1 | 0 | 0 | 0 | 0 | 1,8 | 97,2 | 7 | 1 |
| 9 | 1 | 1,05 | 0 | 0 | 0 | 0 | 97,95 | 6,8 | 7,5 |
| 10 | 1 | 0 | 1,5 | 0 | 0 | 0 | 97,5 | 6,8 | 15 |
| 11 | 1 | 0 | 0 | 6 | 0 | 0 | 93 | 7 | > 10 |
| 12 | 1 | 0 | 0 | 0 | 6 | 0 | 93 | 7 | 5 |

A l'examen des résultats réunis dans le tableau V, il apparaît clairement qu'aucune potentialisation du chlorhydrate de décyloxy-3-hydroxy-2-amino-1-propane comparable à celle obtenue avec le composé E ne se produit avec les autres agents tensio-actifs. Au contraire, deux d'entre eux, à savoir les agents B et C, tendent même à dégrader l'activité bactéricide du chlorhydrate de décyloxy-3-hydroxy-2-amino-1-propane.

La composition conforme à l'invention peut être appliquée dans toutes opérations de désinfection de l'industrie alimentaire, à savoir par exemple

- pour ce qui est de l'industrie de la viande, dans le nettoyage et la désinfection des surfaces telles que murs, sols, salles de découpe, tapis transporteur,
- pour ce qui est de l'industrie des boissons, pour le nettoyage et la désinfection des cuves, fûts et canalisations.

Elle peut également être mise en oeuvre pour le nettoyage et la désinfection du matériel de traite ou dans la prévention de la mammite subclinique chez les ruminants.

Dans ce dernier cas, la composition conforme à l'invention comporte les constituants indiqués plus haut.

Pour montrer l'efficacité de la composition conforme à l'invention sur les germes responsables de la mammite subclinique chez les ruminants par rapport à des compositions dans lesquelles le N,N-diméthyldodécylamine-N-oxyde est remplacé par les deux agents tensio-actifs amphotères A et B identifiés plus haut, on a procédé à la détermination de la CMB des trois compositions correspondantes sur huit souches bactériennes connues dans l'apparition de la mammite.

Les constituants des compositions mises en oeuvre sont:

| | |
|---|---|
| **Formulation M** | |
| Chlorhydrate de décyloxy-3-hydroxy-2-amino-1-propane | 1 |
| Oxyde de dodécyldiméthylamine | 1,8 |
| pH | 7 |
| **Formulation N** | |
| Chlorhydrate de décyloxy-3-hydroxy-2-amino-1-propane | 1 |
| Cocoamidopropyl-bétaïne | 1,4 |
| pH | 7 |
| **Formulation P** | |
| Chlorhydrate de décyloxy-3-hydroxy-2-amino-1-propane | 1 |
| Cocoamidopropylhydroxysultaïne | 2 |
| pH | 7. |

Ces trois formulations comportent en plus des agents émollients, épaississants et colorants de même nature et dans les mêmes proportions.

On a également testé une composition de référence R ne comportant que 1% (P/V) de chlorhydrate de décyloxy-3-hydroxy-2-amino-1-propane et dépourvue d'agents émollients, épaississants et colorants.

Les résultats des huit expériences de détermination de la CMB sur les huit espèces bactériennes communément responsables de la mammite subclinique sont réunis dans le tableau VI.

TABLEAU VI

| Formulations | C M B (% V/V) | | | | | | | |
|---|---|---|---|---|---|---|---|---|
| | S.aureus | E.coli | S.dysgalactiae | St.uberis | St.agalactiae | S.faecalis | P.aeruginosa | Candida albicans |
| R | 7,5 | 2 | 5 | 2,5 | 2,5 | 1 | 1 | 7,5 |
| M | 1 | 1 | 0,5 | 0,25 | 0,25 | 0,5 | 0,5 | 0,5 |
| N | 5 | 2,5 | 2,5 | NT | NT | NT | NT | 5 |
| P | 10 | 2,5 | 2,5 | NT | NT | NT | NT | 5 |

A l'examen des résultats réunis dans le tableau VI, la supériorité de la composition conforme à l'invention apparaît clairement.

En suite de quoi et quel que soit le mode de réalisation adopté, on dispose ainsi d'une composition désinfectante pour l'industrie agro-alimentaire et pour la médecine vétérinaire dont les caractéristiques résultent suffisamment de ce qui précède et qui présente, par rapport à celles qui existent déjà, des

avantages déterminants.

**Revendications**

1. Composition désinfectante caractérisée par le fait qu'elle comporte, en tant que matière active, en combinaison,
   - d'une part, un agent bactéricide de formule:

   $$R - X - CH_2 - CHOH - CH_2NH_2 \quad (I)$$

   dans laquelle
   - X représente -O-, -S-, -NH- ou $-CH_2-$,
   - R représente un radical aliphatique à chaîne droite en $C_5$ à $C_{18}$ et est différent de $C_{12}$ lorsque X est un radical $-CH_2-$,

   ainsi que ses sels, esters et sels d'alcoyle ammonium quaternaire, et,
   - d'autre part, un agent tensio-actif non ionique de formule:

   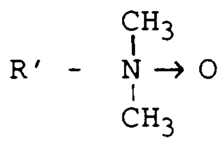

   (II)

   dans laquelle R' représente un radical aliphatique en $C_{12}$ à $C_{16}$,

   étant entendu que ladite composition comporte les agents de formules (I) et (II) en un rapport pondéral II/I compris entre 5 et 0,5.

2. Composition selon la revendication 1, caractérisée par le fait qu'elle comprend en combinaison,
   - à titre d'agent bactéricide, le décyloxy-3-hydroxy-2-amino-1-propane en particulier sous forme de chlorhydrate et,
   - à titre d'agent tensio-actif non ionique, le N,N'-diméthyldodécylamine-N-oxyde.

3. Composition selon l'une des revendications 1 et 2, caractérisée par le fait que l'agent tensio-actif non ionique est un mélange de composés de formule (II) comportant une très forte proportion, notamment supérieure à 65% en poids, de N,N'-diméthyldodécylamine-N-oxyde.

4. Composition selon l'une des revendications 1 à 3, caractérisée par le fait qu'elle contient de 50 à 2% en poids de la combinaison d'agents de formules (I) et (II).

5. Composition selon l'une des revendications 1 à 4, caractérisée par le fait qu'elle contient les agents de formules (I) et (II) en un rapport pondéral II/I compris entre 3 et 1.

6. Composition selon l'une des revendications 1 à 5, caractérisée par le fait qu'elle peut contenir, outre la combinaison des agents de formules (I) et (II),
   - au moins un agent tensio-actif,
   - au moins un agent émollient à effet nourrissant, adoucissant et cicatrisant et
   - au moins un agent filmogène et/ou épaississant, la formulation pouvant être complétée par de l'eau et/ou par au moins un alcool et/ou par un colorant et/ou par un parfum.

7. Application de la composition selon l'une des revendications 1 à 6, à la désinfection dans l'industrie laitière, à la ferme, dans les abattoirs, dans les installations de fabrication de charcuterie et de boissons.

**Claims**

1. Disinfectant composition characterized by the fact that it comprises, as active material, in combination,
   - on the one hand, a bactericidal agent of formula:

R - X - $CH_2$ - CHOH - $CH_2NH_2$ (I)

in which

- X represents -O-, -S-, -NH- or -$CH_2$-,
- R represents an aliphatic $C_5$ to $C_{18}$ straight chain and is different from $C_{12}$ when X is a radical -$CH_2$-,

as well as its salts, esters and quaternary ammonium alkyl salts,
and,

- on the other hand, a non ionic surfactive agent having the formula:

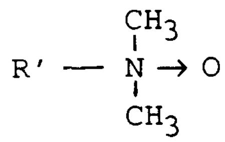 (II)

in which R' represents an aliphatic $C_{12}$ to $C_{16}$ radical,
it being understood that said composition comprises the agents of formulae (I) and (II) in a weight ratio II/I comprised between 5 and 0.5.

2. Composition according to claim 1, characterized by the fact that it comprises in combination,
   - as a bactericidal agent, the 3-decyloxy-2-hydroxy-1-amino-propane in particular under the form of chlorhydrate and,
   - as a non ionic surfactive agent, the N,N'-dimethyldodecylamine-N-oxide.

3. Composition according to one of claims 1 and 2, characterized by the fact that the non ionic surfactive agent is a mixture of compounds of formula (II) comprising a very great proportion, namely higher than 65% by weight, of N,N'-dimethyldodecylamine-N-oxide.

4. Composition according to one of claims 1 to 3, characterized by the fact that it comprises from 50 to 2% by weight of the combination of the agents of formulae (I) and (II).

5. Composition according to one of claims 1 to 4, characterized by the fact that it comprises the agents of formulae (I) and (II) in a weight ratio II/I comprised between 3 and 1.

6. Composition according to one of claims 1 to 5, characterized by the fact that it may contain, besides the combination of the agents of formulae (I) and (II),
   - at least one surfactive agent,
   - at least one softening agent having a nutritional, sweetening and cicatrizing effect and
   - at least one filmogen and/or thickening agent, it being possible to complement the formulation with water and/or with at least one alcohol and/or with a dye and/or with a perfume.

7. Use of the composition according to one of claims 1 to 6, for the disinfection in dairy industry, in farms, in slaughterhouses, in installations for the preparation of cook pork meats and of beverages.

## Patentansprüche

1. Desinfektionszusammensetzung, dadurch gekennzeichnet, daß sie als Wirkstoff eine Kombination umfaßt von
   - einerseits einem bakteriziden Mittel der Formel:

R - X - $CH_2$ - CHOH - $CH_2NH_2$ (I)

worin

- X -O-, -S-, -NH-, oder -$CH_2$- ist,
- R ein geradkettiger aliphatischer $C_5$ bis $C_{18}$ Rest ist und nicht $C_{12}$ ist, wenn X ein -$CH_2$- Rest ist,

sowie dessen Salze, Ester und quaternäre Ammoniumalkylsalze, und

- andererseits einem nichtionischen oberflächenaktiven Mittel der Formel:

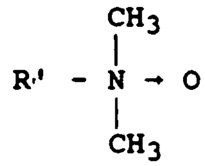

(II)

worin R' ein aliphatischer $C_{12}$ bis $C_{16}$ Rest ist,

wobei verstanden wird, daß die Zusammensetzung die Mittel gemäß der Formeln (I) und (II) in einem Gewichtsverhältnis II/I zwischen 5 und 0,5 umfaßt.

**2.** Zusammensetzung nach Anspruch 1, dadurch gekennzeichnet, daß sie in Kombination umfaßt
- als bakterizides Mittel Decyloxy-3-hydroxy-2-amino-1-propan, insbesondere in Form des Chlorhydrats,
- als oberflächenaktives Mittel N,N'-Dimethyldodecylamin-N-oxid.

**3.** Zusammensetzung nach einem der Ansprüche 1 und 2, dadurch gekennzeichnet, daß das nichtionische oberflächenaktive Mittel ein Gemisch von Verbindungen gemäß Formel (II) ist, welches einen sehr hohen Anteil, insbesondere mehr als 65 Gew.-%, N,N'-Dimethyldodecylamin-N-oxid umfaßt.

**4.** Zusammensetzung nach einem der Ansprüche 1 bis 3, dadurch gekennzeichnet, daß sie 50 bis 2 Gew.-% der Kombination von Mitteln gemäß der Formeln (I) und (II) enthält.

**5.** Zusammensetzung nach einem der Ansprüche 1 bis 4, dadurch gekennzeichnet, daß sie die Mittel gemäß der Formeln (I) und (II) in einem Gewichtsverhältnis II/I zwischen 3 und 1 enthält.

**6.** Zusammensetzung nach einem der Ansprüche 1 bis 5, dadurch gekennzeichnet, daß sie neben der Kombination von Mitteln gemäß der Formeln (I) und (II) enthalten kann:
- mindestens ein oberflächenaktives Mittel,
- mindestens einen weichmachenden Zusatzstoff mit nährender, mildernder und lindernder Wirkung, und
- mindestens einen Filmbildner und/oder ein Verdickungsmittel,

wobei die Formulierung mit Wasser und/oder mindestens einem Alkohol und/oder einem Farbstoff und/oder einem Duftstoff vervollständigt sein kann.

**7.** Verwendung der Zusammensetzung nach einem der Ansprüche 1 bis 6 zur Desinfizierung in der Milchindustrie, in der Landwirtschaft, in Schlachthöfen, in Herstellungsanlagen für Fleischwaren und Getränke.